# EUROPEAN PATENT SPECIFICATION

(11) **EP 2 814 793 B1**
(45) Date of publication and mention of the grant of the patent: **25.05.2016**
(21) Application number: 13703832.9
(22) Date of filing: 14.02.2013
(51) Int. Cl.: C07C 7/00, C07C 7/04, C07C 7/171, C07C 15/02

(54) **METHOD FOR ISOLATION OF CYMENE**
VERFAHREN ZUR ISOLIERUNG VON CYMEN
PROCÉDÉ D'ISOLEMENT DE CYMÈNE

(30) Priority: 14.02.2012 SE 1250118; 14.02.2012 US 201261598545 P
(43) Date of publication of application: 24.12.2014
(73) Proprietor: Holmen AB, 114 84 Stockholm (SE)
(72) Inventor: BRÜCHER, Jörg, S-892 92 Domsjö (SE); BLOMBERG SAITTON, David, S-892 90 Domsjö (SE)
(74) Representative: Zacco Sweden AB
(86) International application number: PCT/EP2013/052936
(87) International publication number: WO 2013/120930

(56) References cited:
- GB-A- 403 614
- GB-A- 765 752
- US-A- 1 893 802

## Description

### Technical field

The present invention relates to a method for isolation and purification of cymene. In particular it relates to a method for isolation of cymene from a solution comprising cymene and monoterpenes.

### Background

Cymene is a naturally occurring aromatic organic compound which structure consists of a benzene ring substituted with a methyl group and an isopropyl group. The structure of cymene is similar to the numerous monoterpenes containing a cyclohexene or cyclohexadiene ring but in contrast to those and other monoterpenes, cymene is a stable compound not undergoing the typical reactions of terpenes. The most common geometric isomer is p-cymene, in which the alkyl groups are para-substituted. There also exists two less common geometric isomers; o-Cymene, in which the alkyl groups are *ortho*-substituted, and *m*-cymene, in which they are *meta*-substituted. p-cymene and *m*-cymene are valuable base chemicals which for example are used in fragrances, pharmaceuticals, herbicides, dyes, and heat transfer media. One other industrially important use of p-cymene is as a starting material for p- cresol production via the Hock- Lange synthesis pathway (1-4). Furthermore, p-cymene has also been proposed as a suitable ingredient in aviation fuel formulations. Compared to other aromatics used in automotive formulations, such as benzene, toluene or ethyl benzene, cymene has lower toxicity and is degraded easier in both aquatic and terrestrial systems. GB 403 614 discloses a method of isolating cymene from turpentine.

Turpentine from boreal hard- and softwood species is a complex mixture of different monoterpenes with α- pinene, β- pinene and carene as main constituents. As sterical strained, unsaturated hydrocarbons, terpenes are highly reactive easily undergoing rearrangements, dimerisation or trimerisation or oxidation reactions. During the sulphate pulping process, terpenes stay unaltered and are condensed together with methanol from the off- gases. The turpentine is separated from other liquids by decantation, forming the typical crude sulphate turpentine (CST). Dominating impurities in CST are methanol along with organic sulphur compounds, polysulphides, and elementary sulphur. Turpentine is almost insoluble in water and thus CST and other turpentines generally contains only small amounts of water, such as less than 1 %.

Monoterpenes are a class of terpenes that consist of two isoprene units and have the molecular formula C₁₀H₁₆. Monoterpenes may be linear (acyclic) or contain rings. Biochemical modifications such as oxidation or rearrangement produce the related monoterpenoids.

### Summary of the present disclosure

Significant amounts of cymene are formed from the wood terpenes in sulphite pulping processes. The present inventors have also realized that cymene can be produced from other kinds of turpentine, such as for example crude sulphate turpentine. However, isolation of cymene from these kind of starting materials are challenging for several reasons. First of all, both the cymene formed in a sulphite pulping process and the cymene produced from e.g. crude sulphate turpentine generally comprises monoterpenes having a boiling point close to the boiling point of cymene and hence, a mixture of monoterpenes and cymene is hard to separate by conventional distillation processes. Furthermore, these kind of starting materials often comprise quite high concentrations of elementary sulphur (S₈), hydrogen sulphide (H₂S) and other sulphur compounds which further complicate the purification of cymene. For example, at elevated temperatures (and/ or low absolute pressure) elementary sulphur can occur as volatile S₈ which can end up as contaminants in a target chemical stream from a distillation process. H₂S can also easily end up in the target chemical stream.

The present inventors have realized that there is a need for improved methods of isolation of cymene from a solution comprising cymene and monoterpenes. The inventors have solved this problem by designing a novel method for isolation of cymene. The method involves addition of sulphuric acid to a solution comprising cymene and monoterpenes such that the monoterpenes polymerizes into diterpenes, triterpenes and/or other oligoterpenes which have a boiling point considerably higher than the boiling point for cymene. Addition of sulphuric acid further promotes reaction of elementary sulphur, H₂S and other sulphur compounds into polysulphides having a high boiling point. Thereby the present invention provides a novel method where cymene easily can be separated from monoterpenes and sulphur compounds by a simple distilling process wherein the majority of the cymene ends up in the distillate and the majority of the monoterpenes and sulphur compounds remain in the residual fraction.

Thus a first aspect of the invention relates to a method of isolating cymene comprising the following steps:
a) providing a solution comprising cymene and monoterpenes,
b) adding sulphuric acid to the solution in a) such that the concentration of sulphuric acid in the solution is at least 0.5 % (w/w), such as 1-25% (w/w),
c) distilling the solution mixture from step b) such that a target chemical stream enriched in cymene is obtained and separated from a residual stream.

### Brief description of the figures

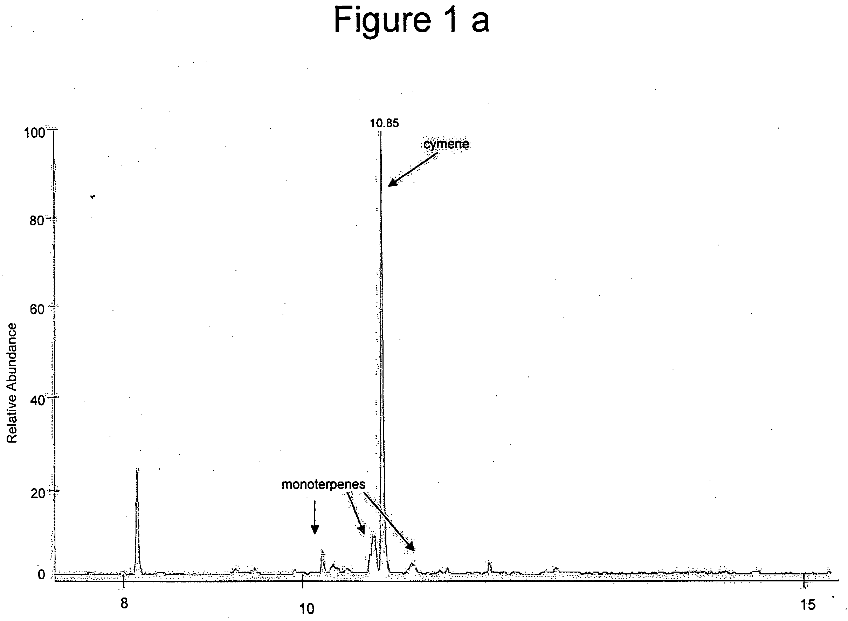

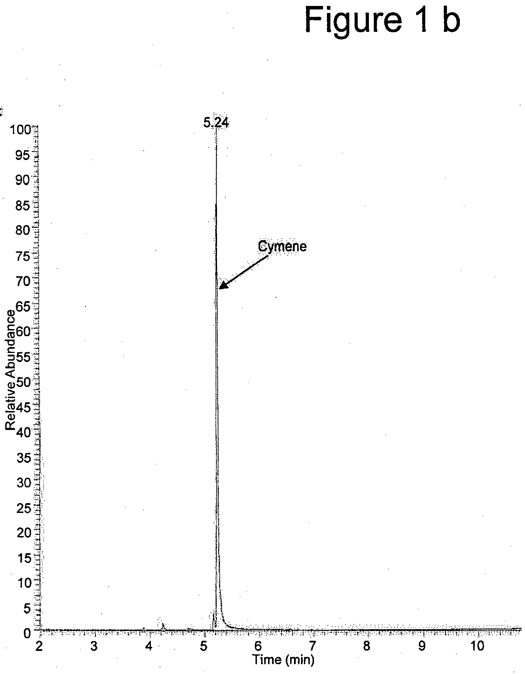

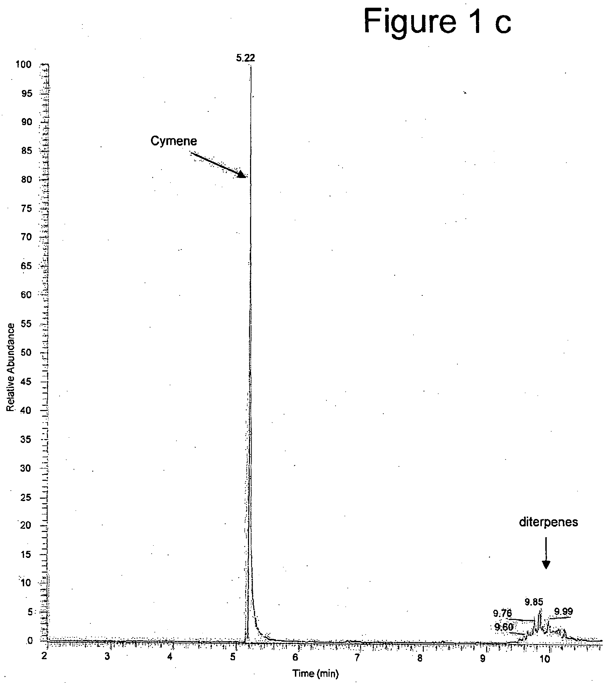

Figure 1 a shows a GC/MS chromatogram of a raw cymene fraction from a sulphite pulp mill comprising about 80 % (w/w) cymene and 15 % (w/w) monoterpenes. The Y-axis shows relative abundance and the X-axis shows the retention time in minutes.
Figure 1b shows a GC/MS chromatogram of the distillate from a distillation process of the raw cymene fraction shown in figure 1a, wherein the distillation process was performed in the presence of about 6.2 % (w/w) sulphuric acid. The Y-axis shows relative abundance and the X-axis shows the retention time in minutes.
Figure 1c shows a GC/MS chromatogram of the residual fraction from a distillation process of the raw cymene fraction shown in figure 1a, wherein the distillation process was performed in the presence of about 6.2 % (w/w) sulphuric acid. The Y-axis shows relative abundance and the X-axis shows the retention time in minutes.

### Detailed description

The present inventors have realized that it is hard to isolate cymene from a mixture of monoterpenes and cymene by conventional distillation processes, since the boiling point of cymene is close to the boiling point of most monoterpenes. For example the boiling point of cymene is about 177 °C and the boiling points of carene, alpha-pinene and beta-pinene, which are the most common monoterpenes present in turpentine, are about 169 °C, 157 °C and 167 °C respectively. The present invention relates to a novel method which solves this problem.

A first aspect of the invention relates to a method of isolating cymene comprising the following steps:
a) providing a solution comprising cymene and monoterpenes,
b) adding sulphuric acid to the solution in a) such that the concentration of sulphuric acid in the solution is at least 0.5 % (w/w), such as 1-25 % (w/w),
c) distilling the solution mixture from step b) such that a target chemical stream enriched in cymene is obtained and separated from a residual stream.

Addition of sulphuric acid to a solution comprising cymene and monoterpenes leads to polymerization of monoterpenes into oligomers (e.g. diterpene and triterpene) having a boiling point which is considerably higher that the boiling point for cymene, such as at least 50 °C higher or at least 100 °C higher.

Even though a sulphuric acid concentration of 0.5 % (w/w) is sufficient for polymerization of at least part of the monoterpenes, higher concentrations are usually preferred to increase the grade of purification. Therefore, in a preferred embodiment sulphuric acid is added to a concentration above 2 % (w/w) more preferably above 3 % (w/w) and most preferably above 5 % (w/w) in step b).

In one embodiment the target chemical stream obtained in step c) comprises at least 75 %, such as at least 80 %, such as at least 90 %, of the cymene present in the solution comprising cymene and monoterpenes provided in step a). In one embodiment at least part of the monoterpenes from step a) react to diterpenes, triterpenes, oligoterpenes and/or polyterpenes in step b) and/or c). In a preferred embodiment the majority, such as at least 50 %, preferably at least 90% of the terpenes in the residual stream in step c) is in the form of diterpenes, triterpenes, oligoterpenes and/or polyterpenes. In one embodiment at least 50 %, preferably at least 90 % of the terpenes in the solution in step a) is in the form of monoterpenes.

It is possible that a portion of the monoterpenes present in the solution obtained in step a) is oxidized into cymene during step b) and or c). However, since the monoterpenes are converted into oligomers during step b) and/or c) the levels of produced cymene during step b) and/or c) will normally be small or even negligible. Therefore, in one embodiment at least 50 %, such as at least 75 %, preferably at least 90 % such as at least 99 % such as all of the cymene molecules present in the target chemical stream obtained in step c) is the same cymene molecules as the cymene molecules present in the solution in step a).

Several industrial relevant sources of cymene comprise not only terpenes but also quite high concentrations of sulphur compounds such as elementary sulphur (S₈) or hydrogen sulphide (H₂S). In conventional distillation processes, the sulphur contaminations further complicates the purification of cymene from these kinds of starting materials. However, the method according to the present invention solves this problem and thus the method is particularly suitable for starting materials having a relatively high concentration of sulphur. Accordingly, in one embodiment the solution comprising cymene and monoterpenes provided in step a) comprises elementary sulphur and/or H₂S and at least part of the elementary sulphur and/or H₂S reacts to form polysulphides in step b) and/or step c). In one embodiment the sulphur content of the solution comprising cymene and monoterpenes provided in step a) is at least 0.1 % (w/w), such as at least 0.5 % (w/w). In one embodiment the solution comprising cymene and monoterpenes provided in step a) is turpentine and in one embodiment the turpentine is crude sulphate turpentine (CST) or turpentine from a sulphite pulping process (sulphite turpentine) or turpentine from a thermo mechanical pulping process (TMP turpentine). CST and sulphite turpentine generally comprise high levels of sulphur and hence the method is particularly suitable for these kinds of turpentine. Hence in a preferred embodiment the solution comprising cymene and monoterpenes provided in step a) is derived from CST or sulphite turpentine. In one embodiment the solution comprising cymene and monoterpenes provided in step a) is sulphite turpentine

The present inventors have also realized that cymene can be produced from a solution comprising cyclic monoterpenes. Said method comprises the following steps:
i) providing a solution comprising cyclic monoterpenes having the formula C₁₀H₁₆,
ii) addition of a catalyst comprising Fe³⁺ and/ or Fe²⁺ ions,
iii) addition of sulphur dioxide to the solution,
iv) incubation of the solution mixture from step iii) for a reaction time at a reaction temperature such that at least part of the cyclic monoterpenes are oxidized to cymene such that a solution comprising cymene is obtained.

Therefore, in one embodiment the solution comprising cymene and monoterpenes provided in step a) is provided by a method comprising the steps of:
i) providing a solution comprising cyclic monoterpenes having the formula C₁₀H₁₆,
ii) addition of a catalyst comprising Fe³⁺ and/ or Fe²⁺ ions,
iii) addition of sulphur dioxide to the solution,
iv) incubation of the solution mixture from step iii) for a reaction time at a reaction temperature such that at least part of the cyclic monoterpenes are oxidized to cymene such that a solution comprising cymene is obtained. In one embodiment the solution comprising cyclic monoterpenes having the formula C₁₀H₁₆ is CST.

Sometimes it is preferred to perform the distilling process at a lower temperature. This can be achieved if the pressure is decreased in the distilling process. Thus, in one embodiment the distilling in step c) is performed at a distilling temperature below 170 °C, such as below 120 °C, such as 20°C - 120°C. In one embodiment the distilling in step c) is performed at a reduced pressure, such as at a pressure below 101 kPa, such as at a pressure between 1-50 kPA such as at a pressure between 5-10 kPa.

The cymene in the target chemical stream obtained in step c) may contain water and small amounts of sulphur compounds. The cymene in the target chemical stream can be further purified by removal of water and sulphur contents. This can be achieved by addition of a dehydrating agent. Therefore, in one embodiment the method further comprises a step d) removing water from the target chemical stream obtained in c) by addition of a dehydrating agent. In one embodiment the dehydration agent is selected from a substance comprising CaO, anhydrous MgSO₄, anhydrous Na₂SO₄ and/or anhydrous CaCl₂. In a preferred embodiment the dehydration agent comprises CaO. In one embodiment the CaO in added to a concentration of 1-25 % (w/w), such as 2-10 % (w/w).

In one embodiment at least part of the monoterpenes in step a) is a cyclic monoterpene. In one embodiment at least part of the terpenes in step a) is α-pinene, β-pinene, carene, sabinene, α-thujene, β-thujene and/or limonene. Turpentine from boreal hard- and softwood species is a complex mixture of different terpenes with α- pinene, β- pinene and carene as main constituents. Therefore, in a preferred embodiment at least part of the monoterpenes in step a) is α-pinene, β-pinene, and/or carene, and in a most preferred embodiment the majority of the monoterpenes in step a) are α-pinene, β-pinene, and/or carene. In one embodiment at least part of the cymene in step a) is p-cymene.

The polymerized monoterpens have a high viscosity which gives the obtained residual stream a tar-like appearance and makes it solid at room temperature. If the concentration of monoterpens in the solution comprising cymene and monoterpene obtained in step a) is too high, cymene can get stuck in the formed tar-like substance during step b) and or c). Thus, in a preferred embodiment the concentration of monoterpenes in the solution comprising cymene and monoterpenes provided in step a) is below 70 % (w/w), preferably below 50 % (w/w). In another embodiment the concentration of cymene in the solution comprising cymene and monoterpenes provided in step a) is at least 30 % (w/w), preferably at least 50 % (w/w).

### Examples

100 g of sulphuric acid was added to 1.5 litres of raw cymene, obtained form a sulphite pulp mill. This corresponds to a sulphuric acid concentration of about 6.2 % (w/w). The raw cymene comprised 80 % (w/w) cymene 15 % (w/w) monoterpenes, 2 % (w/w) water and a total sulphur content of 3 % (w/w). The temperature of the resulting raw cymene/sulphuric acid mixture spontaneously raised to about 75 °C and the mixture was thereafter further heated to 110 °C for 10 minutes. After cooling to 70 °C, the mixture was distilled at a pressure of 50 hPa, using a 60 cm Vigreux column. After this distillation the cymene concentration in the obtained distillate was 98 % (w/w) and the yield in distillation process was 80 %. The concentration of contaminating monoterpenes in the distillate was 1.4 % (w/w). To remove traces of water and sulphur, the distillate was further treated with 5 % (w/w) calcium oxide (CaO). After this treatment the total sulphur content was 0.06 % (w/w) and the water content was 0.01 % (w/w). Chromatograms of the raw cymene, the distillate and the residual fraction are shown in figure 1. As evident from these chromatograms, the majority of the terpenes present in the raw cymene are in the form of monoterpenes whereas the majority of the terpenes in the residual fraction are in the form of diterpenes.

Measurements of cymene, monoterpenes sulphur and water concentrations were preformed by MoRe Research AB, Sweden. The concentrations of cymene and monoterpenes were measured by GC/MS; the water concentration was measured using Karl Fischer titration and the concentration of total sulphur was measured using SCAN CM 57-99 (MoRe Research AB), a method based on Schöniger combustion and wherein the end-determination is based on ion chromatography.

### REFERENCES

1. Ito et al., Hydrocarb. Process. 52 (8) (1973), 89.
2. Welstead et al., Encyclopedia Chem. Technol. 9 (1978), 544.
3. Derfer et al., Encyclopedia Chem. Technol. 22 (1978), 709.
4. Barman et al., Chemical Engineering Journal 114 (2005).

## Claims

1. A method of isolating cymene comprising the following steps:
a) providing a solution comprising cymene and monoterpenes,
b) adding sulphuric acid to the solution in a) such that the concentration of sulphuric acid in the solution is at least 0.5 % (w/w), such as 1-25% (w/w), preferably 3-25%,
c) distilling the solution mixture from step b) such that a target chemical stream enriched in cymene is obtained and separated from a residual stream.

2. Method according to claim 1 wherein at least part of the monoterpenes from step a) react to diterpenes, triterpenes, oligoterpenes and/or polyterpenes in step b) and/or c).

3. Method according to any one of the previous claims wherein the majority, such as at least 50%, preferably at least 90% of the terpenes in the residual stream in step c) is in the form of diterpenes, triterpenes, oligoterpenes and/or polyterpenes.

4. Method according to any one of the previous claims wherein the majority, such as at least 50%, preferably at least 90% of the terpenes in the solution in step a) is in the form of monoterpenes.

5. Method according to any one of the previous claims wherein the distilling in step c) is performed at a reduced pressure, such as at a pressure below 101 KPa, such as at a pressure between 1-50 kPA such as at a pressure between 5-10 KPa.

6. Method according to any one of the previous claims wherein the distilling in step c) is performed at a distilling temperature below 170 °C such as below 120°C.

7. Method according to any one of the previous claims further comprising a step
d) removing water from the target chemical stream obtained in c) by addition of a dehydrating agent.

8. Method according to claim 7 wherein the dehydration agent is selected from a substance comprising CaO, anhydrous MgSO₄, anhydrous Na₂SO₄ and/or anhydrous CaCl₂.

9. Method according to claim 8 wherein the dehydration agent comprises CaO.

10. Method according to claim 9 wherein the CaO in added to a concentration of 1-25 % (w/w), such as 2- 10 % (w/w).

11. Method according to any one of the previous claims wherein the solution in step a) comprises elementary sulphur and/or H₂S and wherein at least part of the elementary sulphur and/or H₂S reacts to form polysulphides in step b) and/or c).

12. Method according to any one of the previous claims wherein the solution comprising cymene and monoterpenes provided in step a) has a sulphur content above 0.1 % (w/w).

13. Method according to any one of the previous claims wherein the concentration of cymene in the solution comprising cymene and monoterpenes provided in step a) is at least 30 % (w/w), preferably at least 50 % (w/w).

14. Method according to any one of the previous claims wherein the concentration of monoterpenes in the solution comprising cymene and monoterpenes provided in step a) is below 70 % (w/w), preferably below 50 % (w/w).

## Patentansprüche

1. Verfahren zur Isolierung von Cymol, umfassend die folgenden Schritte:
a) Bereitstellen einer Lösung umfassend Cymol und Monoterpene,
b) Zugabe von Schwefelsäure zur Lösung in a), so dass die Konzentration von Schwefelsäure in der Lösung zumindest 0,5 % (Gew./Gew.), wie beispielsweise 1-25 % (Gew./Gew.), vorzugsweise 3-25 % (Gew./Gew.), beträgt.
c) Destillieren der Lösungsmischung des Schritts b), so dass ein mit Cymol angereicherter chemischer Zielstrom erhalten und von einem residualen Strom getrennt wird.

2. Verfahren nach Anspruch 1, wobei zumindest ein Teil der Monoterpene des Schritts a) auf Diterpene, Triterpene, Oligoterpene und/oder Polyterpene im Schritt b) und/oder c) reagiert.

3. Verfahren nach einem der vorgehenden Ansprüche, wobei die Mehrzahl, wie beispielsweise zumindest 50 %, vorzugsweise zumindest 90 % der Terpene in dem residualen Strom im Schritt c) in Form von Diterpenen, Triterpenen, Oligoterpenen und/oder Polyterpenen vorliegen.

4. Verfahren nach einem der vorgehenden Ansprüche, wobei die Mehrzahl, wie beispielsweise zumindest 50 %, vorzugsweise zumindest 90 % der Terpene in der Lösung im Schritt a) in Form von Monoterpenen vorliegen.

5. Verfahren nach einem der vorgehenden Ansprüche, wobei das Destillieren im Schritt c) bei einem reduzierten Druck, wie beispielsweise bei einem Druck unter 101 KPa, wie beispielsweise bei einem Druck von 1-50 kPA, wie beispielsweise bei einem Druck von 5-10 KPa, durchgeführt wird.

6. Verfahren nach einem der vorgehenden Ansprüche, wobei das Destillieren im Schritt c) bei einer Destillationstemperatur unter 170 °C, wie beispielsweise unter 120 °C, durchgeführt wird.

7. Verfahren nach einem der vorgehenden Ansprüche, welches ferner den folgenden Schritt umfasst:
d) Entfernen von Wasser aus dem in c) erhaltenen chemischen Zielstrom durch die Zugabe eines Entwässerungsmittels.

8. Verfahren nach Anspruch 7, wobei das Entwässerungsmittel aus einer Substanz umfassend CaO, wasserfreies MgSO₄, wasserfreies Na₂SO₄ und/oder wasserfreies CaCl₂ ausgewählt ist.

9. Verfahren nach Anspruch 8, wobei das Entwässerungsmittel CaO umfasst.

10. Verfahren nach Anspruch 9, wobei das CaO in einer Konzentration von 1-25 % (Gew./Gew.), wie beispielsweise 2-10 % (Gew./Gew.), zugegeben wird.

11. Verfahren nach einem der vorgehenden Ansprüche, wobei die Lösung im Schritt a) Elementarschwefel und/oder H₂S umfasst, und wobei zumindest ein Teil des Elementarschwefels und/oder des H₂S reagiert, um Polysulfide im Schritt b) und/oder c) zu bilden.

12. Verfahren nach einem der vorgehenden Ansprüche, wobei die im Schritt a) bereitgestellte Lösung umfassend Cymol und Monoterpene einen Schwefelgehalt von über 0,1 % (Gew./Gew.) aufweist.

13. Verfahren nach einem der vorgehenden Ansprüche, wobei die Konzentration von Cymol in der im Schritt a) bereitgestellten Lösung umfassend Cymol und Monoterpene zumindest 30 % (Gew./Gew.), vorzugsweise zumindest 50 % (Gew./Gew.), beträgt.

14. Verfahren nach einem der vorgehenden Ansprüche, wobei die Konzentration von Monoterpenen in der im Schritt a) bereitgestellten Lösung umfassend Cymol und Monoterpene weniger als 70 % (Gew./Gew.), vorzugsweise unter 50 % (Gew./Gew.), beträgt.

## Revendications

1. Procédé d'isolement de cymène, comprenant les étapes consistant à:
a) fournir une solution comprenant cymène et monoterpènes,
b) ajouter un acide sulfurique à la solution en a) si bien que la concentration en acide sulfurique dans la solution est d'au moins 0,5% en poids, telle que 1 à 25% en poids, de préférence de 3 à 25%,
c) distiller le mélange de la solution issu de l'étape b) si bien qu'un courant chimique cible enrichie en cymène est obtenu et séparé d'un courant résiduel.

2. Procédé selon la revendication 1, dans lequel au moins une partie des monoterpènes de l'étape a) réagit à diterpènes, triterpènes, oligoterpenes et/ou polyterpènes à l'étape b) et/ou c).

3. Procédé selon l'une quelconque des revendications précédentes, dans lequel la majorité, par exemple au moins 50%, de préférence au moins 90% des terpènes dans le courant résiduaire à l'étape c) est sous la forme de diterpènes, triterpènes, oligoterpènes et/ou polyterpènes.

4. Procédé selon l'une quelconque des revendications précédentes, dans lequel la majorité, par exemple au moins 50%, de préférence au moins 90% des terpènes dans la solution de l'étape a) est sous la forme de monoterpènes.

5. Procédé selon l'une quelconque des revendications précédentes, dans lequel la distillation à l'étape c) est effectuée à une pression réduite, par exemple à une pression inférieure à 101 KPa, par exemple à une pression comprise entre 1 à 50 kPa, par exemple à une pression comprise entre 5 à 10 kPa.

6. Procédé selon l'une quelconque des revendications précédentes, dans lequel la distillation à l'étape c) est effectuée à une température de distillation inférieure à 170 °C, telle qu'inférieure à 120°C.

7. Procédé selon l'une quelconque des revendications précédentes, comprenant en outre l'étape consistant à:
d) éliminer de l'eau du courant chimique cible obtenue en c) par addition d'un agent déshydratant.

8. Procédé selon la revendication 7, dans lequel l'agent de déshydratation est choisi d'une substance comprenant CaO, MgSO₄ anhydre, Na₂SO₄ anhydre et/ou CaCl₂ anhydre.

9. Procédé selon la revendication 8, dans lequel l'agent de déshydratation comprend CaO.

10. Procédé selon la revendication 9, dans lequel CaO est ajouté à une concentration de 1-25 % en poids, telle que 2-10 % en poids.

11. Procédé selon l'une quelconque des revendications précédentes, dans lequel la solution de l'étape a) comprend du soufre élémentaire et/ou H₂S et dans lequel au moins une partie du soufre élémentaire et/ou H₂S réagit pour former des polysulfures de l'étape b) et/ou c).

12. Procédé selon l'une quelconque des revendications précédentes, dans lequel la solution comprenant cymène et monoterpènes fournie dans l'étape a) présente une teneur en soufre supérieure à 0,1% en poids.

13. Procédé selon l'une quelconque des revendications précédentes, dans lequel la concentration de cymène dans la solution comprenant cymène et monoterpènes fournie dans l'étape a) est au moins 30% en poids, de préférence au moins 50% en poids.

14. Procédé selon l'une quelconque des revendications précédentes, dans lequel la concentration de monoterpènes dans la solution comprenant cymène et monoterpènes fournie dans l'étape a) est inférieure à 70% en poids, de préférence inférieure à 50% en poids.
